# EUROPÄISCHE PATENTANMELDUNG

(11) **EP 0 532 143 A1**
(43) Veröffentlichungstag der Anmeldung: **17.03.1993**
(21) Anmeldenummer: 92250256.2
(22) Anmeldetag: 14.09.1992
(51) Int. Cl.: A61N 1/34

(54) **Neurostimulator**

(30) Priorität: 12.09.1991 DE 4130597
(71) Anmelder: BIOTRONIK Mess- und Therapiegeräte GmbH & Co Ingenieurbüro Berlin, D-12359 Berlin (DE)
(72) Erfinder: Schaldach, Max, Prof. Dr., W-8520 Erlangen (DE); Hutten, Helmut, Prof. Dr., A-8010 Graz (AT)
(74) Vertreter: Christiansen, Henning, Dipl.-Ing.

(57) **Zusammenfassung**

Neurostimulator zur Erzeugung von Stimulationsimpulsen für das zentrale oder periphere Nervensystem, insbesondere zur Schmerzbekämpfung im Bereich des Rückenmarks, wobei ein Impulsgenerator (15), dessen Ausgang mit Stimulationselektroden (20) verbunden ist, mittels einer geeigneten Steuerschaltung (1) intervallweise aktivierbar ist, bei dem die Stimulationsimpulse in periodischen Intervallen erzeugt werden, wobei die Aktivitätsperiode im wesentlichen der Wirkdauer - entsprechend der biologischen Halbwertszeit - der körpereigenen Wirkstoffe (Neurotransmitter) entspricht und die jeweilige Ruhepause derjenigen Zeitdauer entspricht, welche die körpereigenen Wirkstoffe benötigen, um sich für eine entsprechende Aktivitätsperiode zu regenerieren.

## Beschreibung

Die Erfindung betrifft einen Neuro-Stimulator der im Oberbegriff des Anspruchs 1 angegebenen Art.

Ein Neuro-Stimulator bringt bei der Behandlung, insbesondere, wenn eine medikamentöse Therapie nicht ausreicht und chirurgische Eingriffe mit einem hohen Risiko behaftet sind, Erfolge bei Patienten, die aufgrund von peripheren Durchblutungsstörungen unter einem nächtlichen Ruheschmerz leiden. Die bei der Rückenmarkstimulation zu beseitigenden refektorischen Schmerzen treten in den sogenannten Headschen Zonen auf, die ihre sensiblen Fasern aus dem selben Rückenmarksegment beziehen, wie die erkrankten Organe. Die Elektrostimulation soll in den Headschen Zonen empfunden werden. Die Stimulation der Neurofunktionen wird nicht nur zur Schmerzunterdrückung, sondern auch zur Stimulation der Durchblutungssteigerung in den erkrankten Organen eingesetzt.

Ein Problem der implantierten Geräte ist die sich allmählich erschöpfende Energieversorgung. Auch mit Langzeitbatterien sind solche Geräte nur einige Zeit bei gepulster bzw. burstförmigen Stimulation funktionsfähig. Somit kommen bekannte Geräte nicht ohne externe Energiequelle aus.

Auch ein Neuro-Stimulator mit einem externem Sender und mit einem implantierbaren Empfänger, mit einer auf die Haut aufklebbaren Antenne ist aus dem Stand der Technik bekannt. In der postoperativen Phase wird zur Schmerzminderung entweder mit Dauerstrichstimulation oder mit Burststimulation behandelt, wobei über das Patientengerät mit dem Sender die Amplitude und die Dauer der Reizimpulse einstellbar sind. Die Schmerzminderung läßt zum Beispiel bei Gewöhnung allmählich nach, so daß die Dosis gesteigert werden muß. Auch ist es üblich, daß der Patient entscheidet, wie lange er pulsen will. Bei nächtlichem Ruheschmerz wird somit die Nachtruhe immer wieder unterbrochen und es wäre somit wünschenswert, wenn eine automatische Stimulation erfolgen könnte, ohne Störungen bzw. Überdosierung.

So ist bereits ein implantierbarer Neurostimulator mit programmierbarer Logik im implantierbarem Empfänger zur Reizimpulserzeugung und mit implantierbarer Mehrfachelektrode bekannt, der mit einem extrakorporalen Patientengerät zur Stimulation zusammenwirkt, daß in größeren Abständen wahlweise mit einem Programmiergerät in Verbindung gebracht werden kann (M. Schaldach, H. Hutten, J. Jirmann, U. Krainick: Implantierbarer Neurostimulator mit programmierbarer Logik, Biomedizinische Technik 35, Ergänzungsband 2, Seiten 138 bis 140). Mit dem Programmiergerät werden alle Betriebsparameter verändert und dann über eine geeignete Schnittstelle in den Speicher des Patientengerätes übernommen.

Nach dem Einschalten überträgt das Patientengerät drahtlos alle individuell angepaßten Betriebsparameter - wobei der Patient nur die Amplitude und die Dauer des Reizimpulses in bestimmten Grenzen variieren kann - und außerdem auch die zum Betrieb erforderliche Energie auf das Implantat, daß eine Schaltung zur Erzeugung einer geregelten Versorgungsspannung aus dem empfangenen Träger der mit Unterspannungserkennung enthält. Das Implantat weist eine Schaltung zum Empfang, zur Demodulation und Umsetzung des unmittelbar vor dem Stimulationsimpuls gesendeten Elektrodenauswahlwortes in ein 8-bit Datenwort auf, welches nach einer Überprüfung an den CMOS - Elektrodenauswahlschalter weitergeleitet wird. Der empfangene Simulationsimpuls wird nach Demodulation und Verstärkung in einer Analog - Schaltung durch den angewählten CMOS - Elektrodenauswahlschalter an die vorbestimmte Elektrode abgegeben, wobei mit einem zusätzlichen Strommeßwiderstand der Reizstrom gemessen und über einem Meßverstärker verstärkt einer passiven Telemetrieschaltung mitgeteilt werden kann.

Der wirksamen Reizung durch die Stimulationsimpulse steht entgegen, daß durch die Anordnung der Antenne keine ausreichende Senderkopplung bei Bewegungen, insbesondere bei Lageveränderungen des Patienten wahrend des Schlafes, garantiert werden kann. Vielmehr ist eine Rückmeldung vom Implantat zum Patientengerät (passive Telemetrie, durch periodische Verstimmung des Energie Übertragungskreises) erforderlich, durch welches der Patient zur Einhaltung der erforderlichen Bedingung aufgefordert wird.

Bei der Durchführung der Elektrostimulation ist es bereits bekannt, diese durch chemische Wirkstoffe zu unterstützen. Die Wirkstoffe sind üblicher Weise auch durch Injektionen oder durch Pillen u.a. Medikamentenspender oder auch von außen dem Körper des Patienten zuführbar, jedoch kann die Wirkung der Medikamente nur schwer ortlich konzentriert werden. Dazu sind bereits spezielle Plaster zur dosierten Langzeitmedikamentengabe bekannt. Die erzielbaren Effekte sind jedoch nicht geeignet, den Energieverbrauch des Neuro-Stimulators grundlegend zu reduzieren.

Der Erfindung liegt die Aufgabe zugrunde, bei geringen Energieverbrauch einer elektronischen Schaltung der eingangs genannten Gattung ihre Wirkung zu verbessern.

Diese Aufgabe wird mit den kennzeichnenden Merkmalen des Anspruchs 1 gelöst.

Basierend auf dem bekannten allgemeinen Ablauf der Arbeitsweise und der Funktion von Nervenzellen kann in diesen Ablauf steuernd eingegriffen werden, indem durch die Elektrostimulation des zentralen oder peripheren Nervensystems ortlich begrenzt chemische Wirkstoffe freigesetzt werden.

Die Erfindung geht von der Erkenntnis aus, daß im Gegensatz zur Leitung von elektrischen Ladungen (elektrische Erregungsleitung) die Nervenreize im Nervensystem auch durch chemische Übertragungsstoffe (Neurotransmitter) auf chemischem Wege weitergeleitet werden. Neurotransmitter werden an den Endigungen der präsynaptischen Faser synthetisiert und dort in Vesikeln gespeichert. Werden diese bei einem ankommenden Aktionspotential freigesetzt, bewirken sie nach rascher Diffusion durch den synaptischen Spalt eine Potentialveränderung an der postsynaptischen Membran, wodurch die elektrische Weiterleitung bzw. Erregung gesteuert wird. Die präganglionären Neuroren des Para- und des Sympathikus sind beispielsweise cholinerg, auch die postganglionären Neuronen des Parasympathikus sind cholinerg aber die postganglionären Neuronen des Sympathikus sind noradrenerg. Die Inaktivierung erfolgt teils durch chemische Wirkstoffe (bei Acetylcholin durch Cholinesterase) teils durch Wiederaufnahme in die Vesikel (bei Noradrenalin).

Als vorteilhaft hat sich dabei erwiesen, daß Neurotransmitter gezielt auf die Nervenfunktion einwirken und diese so anregen, daß diese, trotz Reduzierung der elektrischen Energie, bevorzugt auf die Elektrostimulation reagieren. Bei der gezielten Stimulierung werden offenbar chemische Wirkstoffe mit längerer biologischer Halbwertszeit freigesetzt, die die Schmerzbahn im Rückenmark blockieren. Durch eine Zeitsteuerung, welche die Höhe der Impulsamplituden und/oder die zeitlichen Abstände zwischen den Impulsen und/oder die Impulsbreite bzw. die Abstände zwischen den Burst sowie den Zeitpunkt und die Dauer der Stimulierung beeinflußt, wird der Energieverbrauch des implantierten Neuro-Stimulators reduziert.

Die Zeitsteuerung der elektronischen Schaltung des Neurostimulators wird dabei der jeweiligen spezifischen biologische Halbwertszeit dieser chemischen Wirkstoffe angepaßt gestaltet, so daß quasi gleichförmig über eine lange Zeit die Wirkung bei minimalem Energieaufwand aufrecht erhalten werden kann.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: ein Blockschaltbild einer elektronischen Schaltung eines Ausführungsbeispiels des erfindungsgemäßen Neurostimulators,
- Figur 2: ein zugehöriges Stimulationsdiagramm sowie
- Figur 3: ein Blockschaltbild eines weiteren Ausführungsbeispiels einer Schaltung für einen Neurosimulatior mit einer Kommunikationseinrichtung.

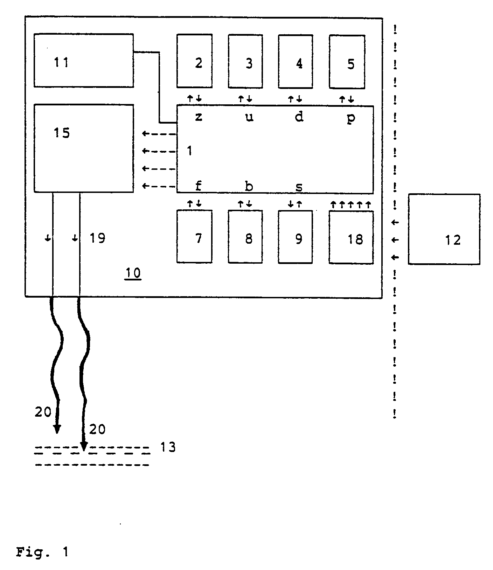

In Figur 1 ist ein Blockschaltbild einer elektronischen Schaltung 10 des Ausführungsbeispiels des erfindungsgemäßen Neurostimulators dargestellt, die eine Zeitsteuerung 1 enthält, an die einen Speicher 2 für den Wert der biologischen Halbwertszeit z, einen Speicher 3 für den Wert der Impulsamplitude u, einen Speicher 4 für den Wert der Einzelimpulsdauer d, einen Speicher 5 für den Wert der Pause zwischen Impulspaketen p, einen Speicher 7 für den Frequenzwert f, einen Speicher 8 für den Wert der Burstbreite b und einen Speicher 9 für die Werte der Betriebsart s. Entsprechend zum Wert der erforderlichen Dosierung werden im Speicher 9 für die Polarität des Impulses oder für den Wechsel der Polarität sowie den Soft-Start Werte s vorgegeben. Über die Zeitsteuerung 1 wird der Ablauf und der Neurostimulationsgenerator 15 so gesteuert, daß weniger Energie, bei örtlicher Anregung der Neuronen durch die Neurostimulation benötigt wird. Durch die Anpassung der Widerholrate der Stimulation an die Halbwertszeit der körpereigenen chemischen Wirkstoffe der Neurotransmitter kann der Abstand zwischen den Phasen der Elektrostimulation vergrößert und damit Energie eingespart werden.

Die Zeitsteuerung 1 kann zur Programmierung über eine Empfangseinheit 18 mit Signalen beaufschlagt werden und gestattet die zeitlichen Abstände zwischen den Phasen der Elektrostimulation entsprechend der biologischen Halbwertszeit der freigesetzten körpereigenen chemischen Wirkstoffe und alle übrigen Parameter ebenfalls programmgemäß zu beeinflussen. Die Programmierung erfolgt in wählbaren Abständen durch eine externe Programmiereinheit 12.

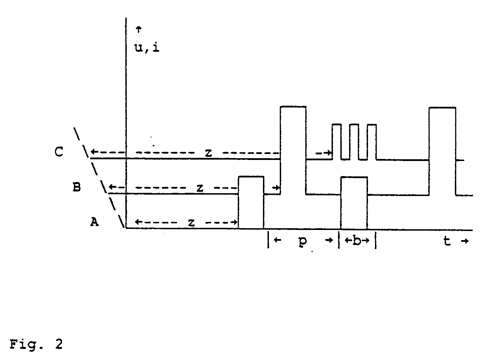

Die Figur 2 zeigt ein typisches Stimulationsdiagramm einer Schaltung gemäß Figur 1. Deutlich sichtbar sind die zeitlichen Phasen der Elektrostimulation von der biologischen Halbwertszeit abhängig, während die aufzuwendende Stimulationsenergie und damit die Dosierung der freigesetzten chemischen Wirkstoffe örtlich verschieden groß ist. Nicht dargestellt ist, wie die einzelnen Zonen abwechselnd aufeinanderfolgend stimulierbar sind.

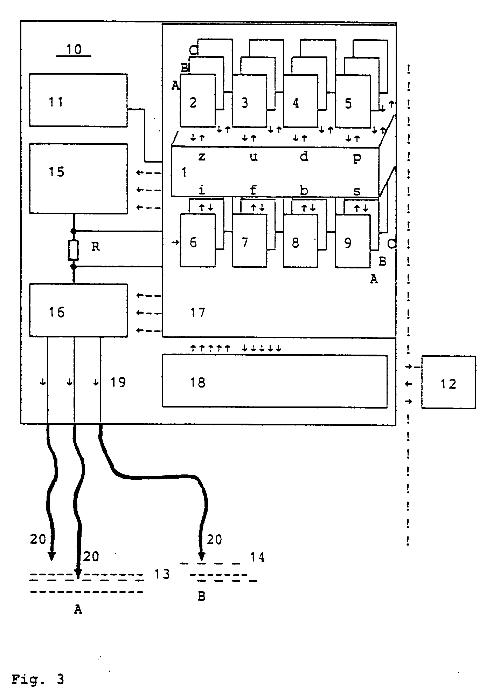

In der in der Figur 3 gezeigten Weiterbildung der Erfindung kann auch bei geigneter Programmiermöglichkeit durch eine erweiterte externe Programmier- und Überwachungseinheit 12, sowohl über den vorgegebenen Abstand zwischen den Phasen der Elektrostimulierung entsprechend der biologischen Halbwertszeit, die örtliche Dosierung der chemischen Wirkstoffe verändert, als auch der erforderliche Dosiermengenwert signalisiert, und somit die nötige Dosiermenge ärztlicherseits kontrolliert werden. Hierzu wird eine Stimulatorsteuerung 17 eingesetzt. Diese ist über Meßleitungen mit dem Strommeßwiderstand R in den Zuleitungen 19 zu den Elektroden 20 verbunden. Der so gemessene aktuelle Stromwert i wird in einen Speicher 6 übernommen und abgespeichert. Durch die Stimulatorsteuerung 17 wird laufend über den Strommeßwiderstand der Strom i und daraus Dosiermengenwert M oder der Gewebewiderstand Rg am Ort der Elektrodenplatzierung bzw. in Verbindung damit der aktuelle Wert z der biologischen Halbwertszeit ermittelt, welche bei Bedarf durch die externe Programmier- und Überwachungseinheit 12 abgefragt bzw. vorgegeben werden. Somit können diesbezügliche Veränderungen erfaßt und für eine erneute Programmierung ausgewertet werden. Die Zeitsteuerung 1 und die zugehörigen Speicher 2 bis 9 sind in Ebenen A bis C entsprechend der Orte der Elektrostimulation bzw. Behandlungsbereiche gegliedert.

In vorteilhafter Weise wird die Zeitsteuerung 1 softwaremäßig durch die einen Mikrorechner enthaltenen Stimulatorsteuerung 7 ausgeführt.

Bei implantierten mehrfachen Elektroden wird die Anschaltung der betreffenden Elektrode 20 über einen Elektrodenauswahlschalter 16 vorgenommen. Die einzelnen Sektionen 13, 14 des Rückenmarks können allein oder einzeln nacheinander gezielt stimuliert werden. Die Wirkung hält darauf hin in den Körperbereichen A, B, C usw. längere Zeit an.

Der Ladezustand der Batterie 11 wird ebenfalls ständig durch die Stimulatorsteuerung 17 überwacht, welche auch die Abstände (Pause) der Impulsgruppen steuert, in welchen der Neurostimulatorgenerator 15 burstförmig Impulse an mindestens eine Elektrode 20 je Stimulierungsort A, B bzw. C abgibt. In diesen Pausen p zwischen den Impulspaketen wird die Spannung der minimal (nur durch die Stimulatorsteuerung 17) belasteten Batterie 11 gemessen. In an sich bekannter Weise kann so der Ladezustand ermittelt, in einem nicht dargestelltem weiteren Speicher zwischengespeichert und bei Bedarf abgefragt werden. Das Gehäuse des implantierbaren Neuro-Stimulators 10 kann als Gegenelektrode ausgebildet sein, so daß nur eine Zuleitung 19 je Elektrode 20 erforderlich ist. Die Polarität der Impulse ist programmierbar und kann alternierend wechseln, wofür der Speicher 9 an die Zeitsteuerung 1 angeschlossen ist. Diese steuert auch eine definierte Anstiegszeit der Impulse zu Beginn und Ende des Impulspaketes.

Zur Kommunikation mit dem implantierten Gerät ist zwar eine externen Programmier - und Überwachungseinheiten 12 bereits an sich bekannt, welche mit einer Empfangs- und Telemetrieeinheit 18 in Verbindung treten können, jedoch arbeitete das Implantat bisher nicht selbsttätig, sondern stand ständig mit dem Patientengerät in Verbindung und mußte auch noch von außen mit Energie versorgt werden.

Außerdem kann nun automatisch auf eine sich verändernde Reizschwelle, die über die Messung des Gewebewiderstandes Rg und aus dem Dosiermengenwert M durch die Zeitsteuerung 1 ermittelt wird, reagiert werden. Die externe Programmier und Überwachungseinheit (12) kann mit der Empfangs- und Telemetrieeinheit (18) des Neuro-Stimulators (10) zu beliebigen Zeitpunkten in Verbindung gebracht werden. In vorteilhafter Weise die Kommunikation über Laserdioden und ein mindestens teilweise lichtdurchlässiges Gehäuse, wenn die externe Programmier - und Überwachungseinheit (12) auf die Haut, unter der sich das Implantat befindet, aufgesetzt wird. In der übrigen Zeit arbeitet der implanierte Neuro-Stimulator selbsttätig und ist aufgrund seiner Batterie 11 autark. Somit entfällt die Abhängigkeit von einer Sender-Empfänger-Kopplung bzw. die Anfälligkeit gegenüber Bewegungen des Patienten.

Der Neurostimulationsgenerator 15, an welchem Elektroden 20 an dem einem Ende einer Leitung 19 angeschlossen sind, wirkt örtlich begrenzt am anderen Ende der Leitung 19, so daß chemische Wirkstoffe mit einer bestimmten spezifischen Halbwertzeit abgegeben werden, die in den Headschen Zonen eine länger anhaltende Wirkung entsprechend der Elektrostimulation ermöglichen. Patientenspezifische Biorhythmen können dabei berücksichtigt werden, so daß zu einer programmierten Zeit, beispielsweise wärend einer Stunde, einmal fünf Minuten burstförmig stimuliert wird und die Wirkung somit optimiert wird und noch länger anhält.

Bei einer tageszyklischen Kopplung wird die Impulsdosis in der Nacht herabgesetzt.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Neurostimulator zur Erzeugung von Stimulationsimpulsen für das zentrale oder periphere Nervensystem, insbesondere zur Schmerzbekämpfung im Bereich des Rückenmarks, wobei ein Impulsgenerator, dessen Ausgang mit Stimulationselektroden verbunden ist, mittels einer geeigneten Steuerschaltung intervallweise aktivierbar ist,
**dadurch gekennzeichnet,**
daß die Stimulationsimpulse in periodischen Intervallen erzeugt werden, wobei die Aktivitätsperiode im wesentlichen der Wirkdauer - entsprechend der biologischen Halbwertszeit - der körpereigenen Wirkstoffe (Neurotransmitter) entspricht und die jeweilige Ruhepause derjenigen Zeitdauer entspricht, welche die körpereigenen Wirkstoffe benötigen, um sich für eine entsprechende Aktivitätsperiode zu regenerieren.

2. Neurostimulator nach Anspruch 1, **dadurch gekennzeichnet,** daß die Intervalldauer und/oder das Verhältnis von Aktivitätsperiode zur Intervalldauer durch Programmierung veränderbar sind.

3. Neurostimulator nach einem der vorangehenden Ansprüche, **dadurch gekennzeichnet,** daß die Programmierung der Intervalldauer und/oder das Verhältnis von Aktivitätsperiode zur Intervalldauer in einem 24h-Zyklus veränderbar sind.

4. Neurostimulator nach einem der vorangehenden Ansprüche, **dadurch gekennzeichnet,** daß bei implantierbarer Ausführung eine interne Energiequelle vorgesehen ist.

5. Neurostimulator nach einem der vorangehenden Ansprüche, **dadurch gekennzeichnet,** daß eine, insbesondere programmierbare, Auswahlschaltung vorgesehen ist, welche die Erzeugung von elektrischen Verbindungen zwischen dem Ausgang des Impulsgenerators und einer oder mehrerer Elektroden in unterschiedlicher Kombination oder Sequenz ermöglicht.

6. Neurostimulator nach einem der vorangehenden Ansprüche, **dadurch gekennzeichnet,** daß die Stimulation in der Aktivitätsperiode burstartig erfolgt.

7. Neurostimulator nach einem der vorangehenden Ansprüche, daß die Impulsamplitude programmierbar ist.

8. Neurostimulator nach einem der vorangehenden Ansprüche, **dadurch gekennzeichnet,** daß die Programmierung eine Steuerschaltung (1) bei Stimulationsbeginn mit einem Wert der biologischen Halbwertszeit z und einem Dosiermengenwert M erfolgt, die in Verbindung mit einer Stimulatorsteuerung (17) den Neurostimulationsgenerator (15) derart steuert, daß die Elektrostimulation des Rückenmarks während der Wirkdauer nach einem zeitlichen Ablauf erfolgt.

9. Neurostimulator nach einem der vorangehenden Ansprüche, **dadurch gekennzeichnet,** daß eine externe Programmier- und Überwachungseinheit (12) mit der Empfangs- und Telemetrieeinheit (18) vorgesehen ist.

10. Neurostimulator nach Anspruch 8, **dadurch gekennzeichnet,** daß die Kommunikation mittels passiver Telemetrie erfolgt.

11. Neurostimulator nach einem der vorangehenden Ansprüche, **dadurch gekennzeichnet,** daß die Steuerschaltung (1) automatisch auf eine sich verändernde Reizschwelle, die über die Messung des Gewebewiderstandes Rg und aus dem Dosiermengenwert M ermittelt wird, reagiert und sich somit an einen patientenspezifischen Biorhythmus anpaßt.
